# EUROPÄISCHE PATENTSCHRIFT

(11) **EP 1 117 676 B1**
(45) Veröffentlichungstag und Bekanntmachung des Hinweises auf die Patenterteilung: **18.08.2004**
(21) Anmeldenummer: 99947385.3
(22) Anmeldetag: 18.09.1999
(51) Int. Cl.: C07J 43/00, C07K 5/065, A61K 31/575

(54) **MIT GALLENSÄUREN VERKNÜPFTE PROPANOLAMINDERIVATE ZUR BEHANDLUNG VON LIPIDSTOFFWECHSELSTÖRUNGEN**
PROPANOLAMINE DERIVATIVES LINKED WITH BILE ACID USED FOR TREATING DISORDERS OF THE LIPID METABOLISM
DERIVES DE PROPANOLAMINE RETICULES AVEC DES ACIDES GALLIQUES, UTILISES POUR TRAITER DES TROUBLES DU METABOLISME LIPIDIQUE

(30) Priorität: 02.10.1998 DE 19845403
(43) Veröffentlichungstag der Anmeldung: 25.07.2001
(73) Patentinhaber: Aventis Pharma Deutschland GmbH, 65929 Frankfurt am Main (DE)
(72) Erfinder: STENGELIN, Siegfried, D-65817 Eppstein (DE); ENHSEN, Alfons, D-64572 Büttelborn (DE); GLOMBIK, Heiner, D-65719 Hofheim (DE); KRAMER, Werner, D-55130 Mainz-Laubenheim (DE); FALK, Eugen, D-60529 Frankfurt am Main (DE)
(86) Internationale Anmeldenummer: PCT/EP1999/006930
(87) Internationale Veröffentlichungsnummer: WO 2000/020437

(56) Entgegenhaltungen:
- EP-A- 0 345 591
- EP-A- 0 489 423
- EP-A- 0 624 593
- EP-A- 0 869 121
- US-A- 5 610 151

## Beschreibung

Die Erfindung betrifft substituierte Propanolaminderivate und deren pharmazeutisch verträgliche Salze und physiologisch funktionelle Derivate.

Es sind bereits mehrere Wirkstoffklassen zur Behandlung von Adipositas und von Lipidstoffwechselstörungen beschrieben worden:
- polymere Adsorber, wie z.B. Cholestyramin
- Benzothiazepine (WO 93/16055)
- Gallensäuredimere und -konjugate (EP 0 489 423)
- 4-Amino-2-ureido-pyrimidin-5-carbonsäureamide (EP 0 557 879)

Der Erfindung lag die Aufgabe zugrunde, weitere Verbindungen zur Verfügung zu stellen, die eine therapeutisch verwertbare hypolipidämische Wirkung entfalten.

Die Erfindung betrifft daher Verbindungen der Formel I, 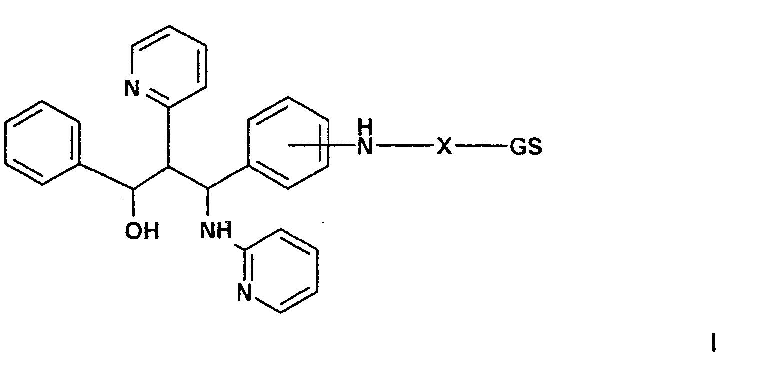 worin bedeuten
- GS: eine Gallensäuregruppe der Formel 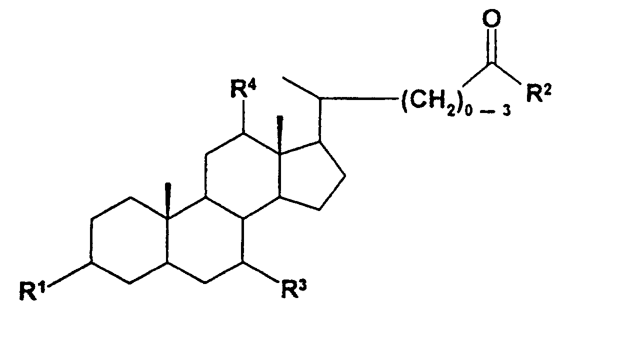
- R¹: eine Bindung zu X, OH;
- R²: eine Bindung zu X, OH, O-(C₁-C₆)-Alkyl , NH-(C₂-C₆)-Alkyl-SO₃H, N(CH₃)-CH₂-CH₂-SO₃H, NH-(C₁-C₆)-Alkyl-COOH; N(CH₃)-(C₁-C₆)-Alkyl-COOH;
mit der Maßgabe, daß R¹ und R² nicht gleichzeitig die folgende Bedeutung haben
- R¹: eine Bindung zu X und
- R²: eine Bindung zu X;
- R³ , R⁴: unabhängig voneinander H, OH;
- X: 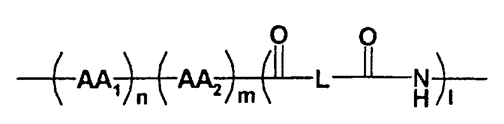 oder eine Bindung;
- l, m, n: unabhängig voneinander 0 oder 1;
- L: (C₁ - C₆)-Alkyl, Phenyl;
- AA₁ , AA₂: unabhängig voneinander einen Aminosäurerest oder einen Aminosäurerest, der einfach oder mehrfach substituiert ist durch eine Aminosäureschutzgruppe;
sowie deren pharmazeutisch verträgliche Salze.

Bevorzugt sind Verbindungen der Formel I, in denen ein oder mehrere Rest(e) die folgende Bedeutung hat bzw. haben:
- GS: eine Gallensäuregruppe der Formel 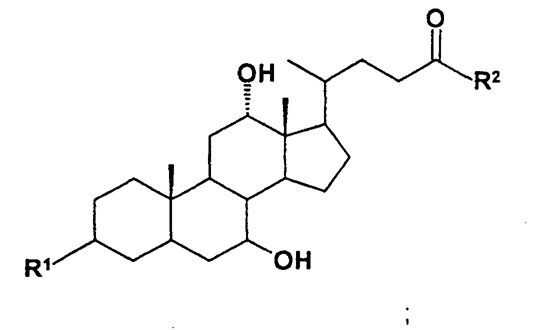
- R¹: eine Bindung zu X, OH;
- R²: eine Bindung zu X, OH, O-(C₁-C₆)-Alkyl, NH-(C₂-C₆)-Alkyl-SO₃H, N(CH₃)-CH₂-CH₂-SO₃H, NH-(C₁-C₆)-Alkyl-COOH; N(CH₃)-(C₁-C₆)-Alkyl-COOH;
mit der Maßgabe, daß R¹ und R² nicht gleichzeitig die folgende Bedeutung haben
- R¹: eine Bindung zu X und
- R²: eine Bindung zu X;
- X: 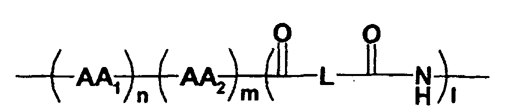
oder eine Bindung;
- l, m, n: unabhängig voneinander 0 oder 1;
- L: (C₁ - C₆)-Alkyl, Phenyl;
- AA₁, AA₂: unabhängig voneinander einen Aminosäurerest oder einen Aminosäurerest, der einfach oder mehrfach substituiert ist durch eine Aminosäureschutzgruppe;
sowie deren pharmazeutisch verträgliche Salze.

Besonders bevorzugt sind Verbindungen der Formel I, in denen ein oder mehrere Rest(e) die folgende Bedeutung hat bzw. haben:
- GS: eine Gallensäuregruppe der Formel 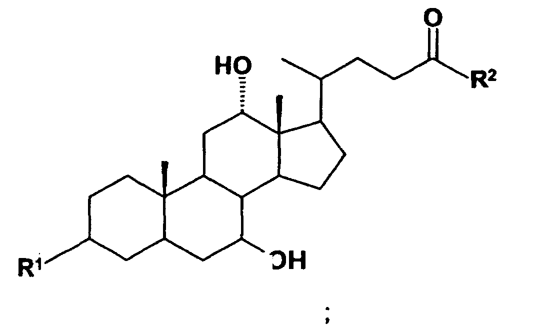
- R¹: eine Bindung zu X, OH;
- R²: eine Bindung zu X, OH, O-(C₁-C₆)-Alkyl, NH-(C₂-C₆)-Alkyl-SO₃H, NH-(C₁-C₆)-Alkyl-COOH;
mit der Maßgabe, daß R¹ und R² nicht gleichzeitig die folgende Bedeutung haben
- R¹: eine Bindung zu X und
- R²: eine Bindung zu X;
- X: 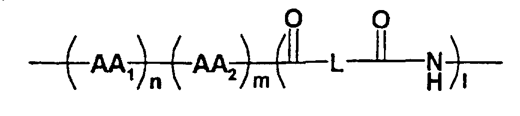 oder eine Bindung;
- l, m, n: unabhängig voneinander 0 oder 1;
- L: (C₁ - C₆)-Alkyl;
- AA₁ , AA₂: unabhängig voneinander einen Aminosäurerest oder einen Aminosäurerest, der einfach oder mehrfach substituiert ist durch eine Aminosäureschutzgruppe;
sowie deren pharmazeutisch verträgliche Salze.

Unter dem Begriff Alkyl werden geradkettige oder verzweigte Kohlenwasserstoffketten verstanden.

Mit dem Begriff Aminosäuren bzw. Aminosäurereste sind die stereoisomeren Formen, d.h. D- oder L-Formen, folgender Verbindungen gemeint:

| | | |
|---|---|---|
| Alanin | Glycin | Prolin |
| Cystein | Histidin | Glutamin |
| Asparaginsäure | Isoleucin | Arginin |
| Glutaminsäure | Lysin | Serin |
| Phenylalanin | Leucin | Threonin |
| Tryptophan | Methionin | Valin |
| Tyrosin | Asparagin | |

| | |
|---|---|
| 2-Aminoadipinsäure | 2-Aminoisobuttersäure |
| 3-Aminoadipinsäure | 3-Aminoisobuttersäure |
| beta-Alanin | 2-Aminopimelinsäure |
| 2-Aminobuttersäure | 2,4-Diaminobuttersäure |
| 4-Aminobuttersäure | Desmosin |
| Piperidinsäure | 2,2-Diaminopimelinsäure |
| 6-Aminocapronsäure | 2,3-Diaminopropionsäure |
| 2-Aminoheptansäure | N-Ethylglycin |
| 2-(2-Thienyl)-glycin | 3-(2-Thienyl)-alanin |
| Penicillamin | N-Methylglycin |
| N-Ethylasparagin | N-Methylisoleucin |
| Hydroxylysin | 6-N-Methyllysin |
| allo-Hydroxylysin | N-Methylvalin |
| 3-Hydroxyprolin | Norvalin |
| 4-Hydroxyprolin | Norleucin |
| Isodesmosin | Ornithin |
| allo-Isoleucin | 11-Aminoundecansäure |

Die Kurzschreibweise der Aminosäuren erfolgte nach der allgemein üblichen Schreibweise (vgl. Schröder, Lübke, The Peptides, Band I, New York 1965, Seiten XXII-XXIII; Houben-Weyl, Methoden der Organischen Chemie, Band XV/1 und 2, Stuttgart 1974). Die Aminosäure D-Asp steht für die D-Form von Asparaginsäure. Peptide sind ihrer chemischen Natur nach Säureamide und zerfallen bei der Hydrolyse in Aminosäuren.

Unter dem Begriff Aminosäureschutzgruppen sind geeignete Gruppen zu verstehen mit denen die funktionellen Gruppen der Seitenketten der Aminosäurereste geschützt sind (siehe z. B. T. W. Greene, P. G. M. Wuts, Protective Groups in Organic Synthesis, 2^{nd} Edition, John Wiley and Sons, New York 1991). Bevorzugte Aminsäureschutzgruppen sind: t-butyloxy-carbonyl (BOC), 9-fluorenylmethoxy-carbonyl (Fmoc), benzyloxy-carbonyl (Z), 2-(3,5-dimethoxyphenyl)prop-2-yloxycarbonyl (Ddz), methyl, t-butyl, trityl, S-t-butyl, t-butylamino-carbonyl.

Die Erfindung betrifft weiterhin Verfahren zur Herstellung von Verbindungen der Formel I, die durch folgende Reaktionsschemata (Schema 1 bis 4) gekennzeichnet sind:

### Verfahren A

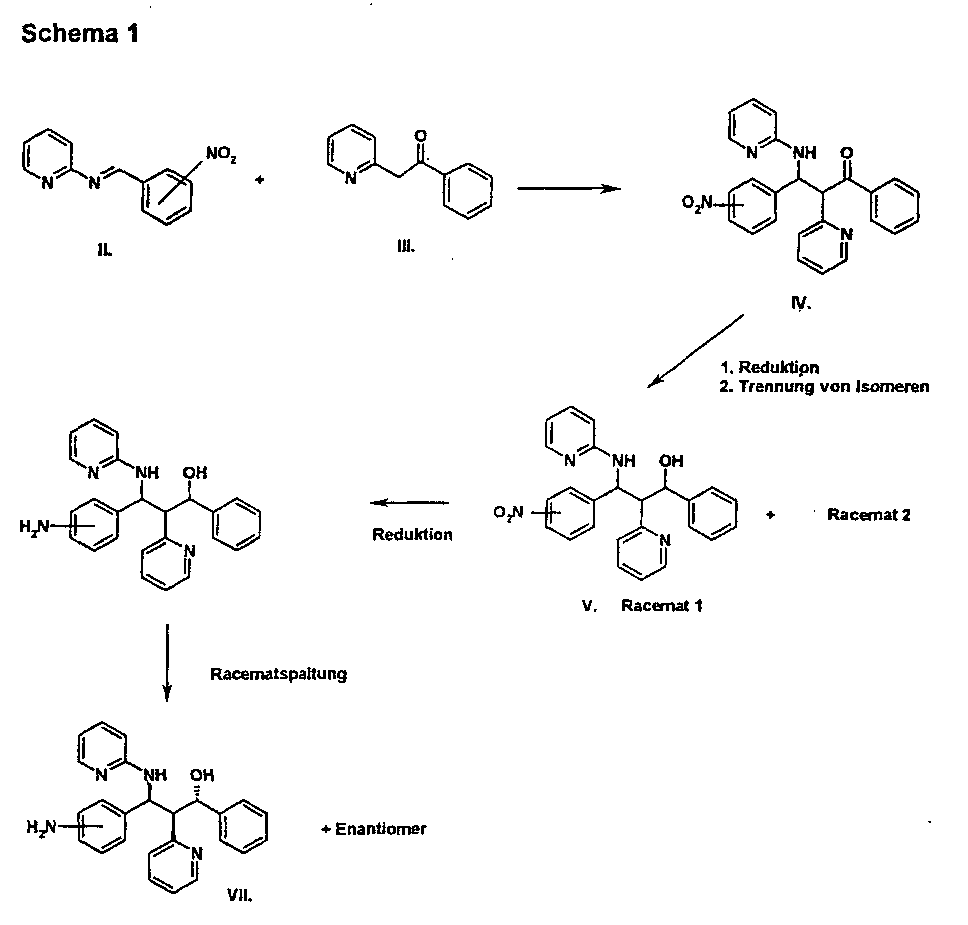

Verbindungen des Typs IV werden erhalten, indem o-, m- oder p-substituierte Imine des Typs II mit dem Keton III zur Reaktion gebracht werden. Die Reaktion kann zum Beispiel durch Mischung der beiden Verbindungen in Substanz, ohne Lösungsmittel, und anschließendem Erhitzen oder in einem geeigneten Lösungsmittel wie Ethanol, Tetrahydrofuran (THF), Toluol, Diglyme oder Tetradecan bei Temperaturen von 20° C bis 150° C durchgeführt werden.
Die Ketoverbindungen des Typs IV werden in einem geeigneten Lösungsmittel, wie z.B. Methanol, THF oder THF/Wasser mit NaBH₄ oder einem anderen geeigneten Reduktionsmittel bei Temperaturen zwischen -30° C und + 40° C zu Hydroxyverbindungen des Typs V reduziert. Bei der Reduktion fallen meist zwei Isomerengemische (Racemate) als Hauptprodukte an. Die unterschiedlichen Racemate können durch fraktionierte Kristallisation oder durch Kieselgelchromatographie voneinander abgetrennt werden. Die Nitrogruppe in Verbindungen des Typs V kann durch bekannte Verfahren, wie z.B. die katalytische Hydrierung mit Pd oder Pd auf Kohle und H₂ in Methanol, reduziert werden.

Die so erhaltenen racemischen Verbindungen des Typs VI kann weiter in ihre Enantiomere aufgetrennt werden. Die Racematspaltung von VI in Enantiomere des Typs VII kann durch Chromatographie über chirales Säulenmaterial oder durch literaturbekannte Verfahren mit optisch aktiven Hilfsreagenzien durchgeführt werden (vgl. J. Org. Chem. 44, 1979, 4891).

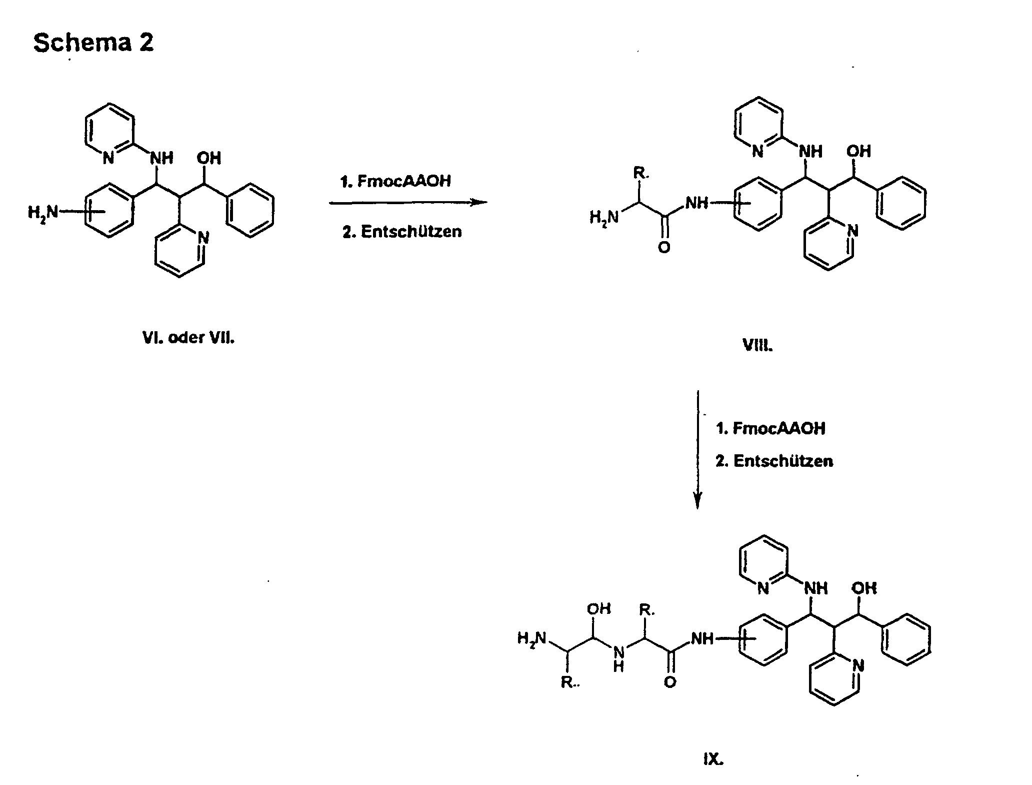

Entsprechend Schema 2 können die aromatischen Amine des Typs VI oder VII (Racemat oder reines Enantiomer) mit einer Aminosäure unter bekannten Standard-Peptikupplungsverfahren zu Derivaten VIII umgesetzt werden. Als Verfahren eignet sich zum Beispiel die Kupplung mit TOTU und Triethylamin in DMF. (Literatur siehe: G. Breipohl, W. König EP 0460446; W. König, G. Breipohl, P. Pokorny, M. Birkner in E. Giralt and D. Andreu (Eds.) Peptides 1990, Escom, Leiden, 1991, 143-145). Die Reste AA₁ und AA₂ haben die unter Formel I genannte Bedeutung. Die Aminofunktion der Aminosäure ist mit einer Schutzgruppe versehen, z.B. Fmoc, die Carbonsäure ist ungeschützt.

Bei Aminosäuren mit funktionellen Gruppen in der Seitenkette sind diese entsprechend geschützt, entweder temporär während der Synthese oder zum Verbleib in den erfindungsgemäßen Verbindungen.

Um zum Derivat VIII zu gelangen wird die Schutzgruppe der Aminofunktion abgespalten, z.B. im Fall von Fmoc in einem Gemisch aus DMF und Piperidin. Dipeptidkonjugate IX werden erhalten, wenn, ausgehend von Verbindungen des Typs VIII, die Reaktionssequenz (a) Kupplung einer Aminosäure (b) Entschützen wiederholt wird.

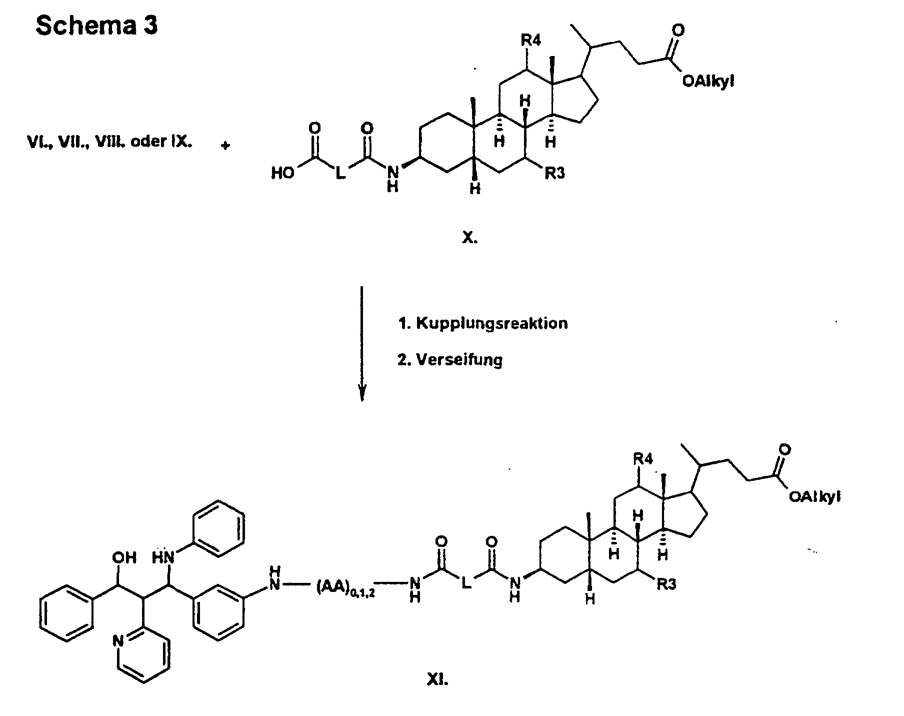

Gallensäurederivate des Typs X können aus 3-Aminogallensäureestern durch Verknüpfung mit Alkyl- oder Aryldicarbonsäuren oder deren Derivaten, wie z.B. Bernsteinsäureanhydrid nach bekannten Verfahren hergestellt werden (z.B. EP 0614908, EP 0489423). Diese Verbindungen (X) werden mit Aminoverbindungen des Typ VI, VII, VIII oder IX nach Standard-Peptidkupplungsverfahren zur Reaktion gebracht. Nach der Kupplungsreaktion werden durch Verseifung der Alkylesterfunktion des Gallensäureteils die Verbindungen des Typs XI erhalten.

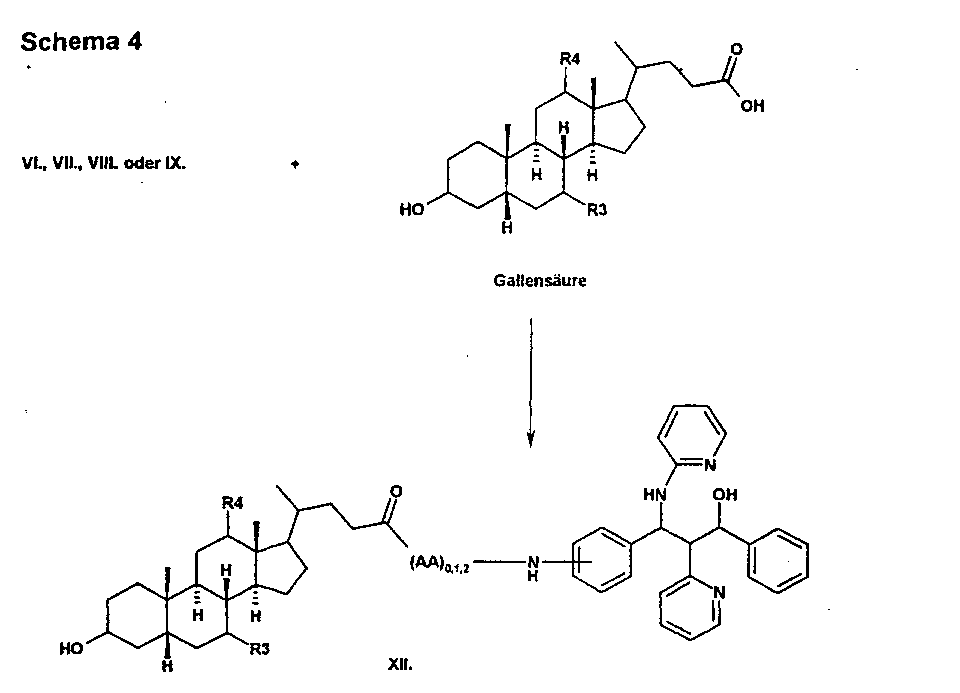

Die Aminoverbindungen des Typs VI, VII, VIII oder XI können mit der Carbonsäurefunktion von Gallensäuren zur Reaktion gebracht. Hier finden ebenfalls bekannte Peptidkupplungsverfahren Verwendung, z.B. Kupplungen in Gegenwart von TOTU und Triethylamin oder mit Dicyclohexylcarbodiimid, Hydroxybenzotriazol und Triethylamin in THF. Nach diesen Verfahren können die Verbindungen des Typs XII erhalten werden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isothion-, Milch-, Lactobion-, Malein-, Apfel-, Methansulfon-, Bernstein-, p-Toluotsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen; z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z. B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Benzothiazepin-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete WirkstoffKonzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Erfindung bezieht sich auf Verbindungen der Formel I, in Form ihrer Racemate, racemischen Mischungen und reinen Enantiomere sowie auf ihre Diastereomere und Mischungen davon.

Die Verbindungen der Formel I und deren pharmazeutisch verträgliche Salze und physiologisch funktionelle Derivate zeichnen sich durch günstige Wirkungen auf den Lipidstoffwechsel aus. Die Verbindungen können allein oder in Kombination mit weiteren lipidsenkenden Wirkstoffen eingesetzt werden. Die Verbindungen eignen sich zur Prophylaxe sowie insbesondere zur Behandlung von Lipidstoffwechselstörungen, insbesondere von Hyperlipidämie dar. Die Verbindungen der Formel I eignen sich ebenfalls zur Beeinflussung des Serumcholesterinspiegels sowie zur Prävention und Behandlung arteriosklerotischer Erscheinungen.
Folgende Befunde belegen die pharmakologische Wirksamkeit der erfindungsgemäßen Verbindungen.

Die biologische Prüfung der erfindungsgemäßen Verbindungen erfolgte durch Ermittlung der Hemmung der [³H]-Taurocholataufnahme in Bürstensaummembranvesikel des Ileums von Kaninchen. Der Hemmtest wurde wie folgt durchgeführt:

### 1. Präparation von Bürstensaummembranvesikeln aus dem Ileum von Kaninchen

Die Präparation von Bürstensaummembranvisikeln aus den Darmzellen des Dünndarm erfolgte mit der sogenannten Mg²⁺-Präzipitationsmethode. Männliche Neuseeland-Kaninchen (2 bis 2,5 kg Körpergewicht) wurden durch intravenöse Injektion von 0,5 ml T61®, einer wäßrigen Lösung von 2,5 mg Tetracain HCl, 100 m Embutramid und 25 mg Mebezoniumjodid getötet. Der Dünndarm wurde entnommen und mit eiskalter physiologischer Kochsalzlösung gespült. Die terminalen 7/10 des Dünndarms (gemessen in oral-rektaler Richtung, d.h. das terminale Ileum, welches das aktive Na⁺-abhängige Gallensäuretransportsystem enthält) wurden zur Präparation der Bürstensaummembranvesikel verwendet. Die Därme wurden in Kunststoffbeuteln unter Stickstoff bei -80°C eingefroren. Zur Präparation der Membranvesikel wurden die eingefrorenen Därme bei 30°C im Wasserbad aufgetaut. Die Mucosa wurde abgeschabt und in 60 ml eiskaltem 12 mM Tris/HCl-Puffer (pH 7,1)/300 mM Mannit, 5 mM EGTA/10 mg/l Phenylmethyl-sulfonylfluorid/1 mg/l Trypsin Inhibitor v. Sojabohnen (32 U/mg)/0,5 mg/l Trypsin Inhibitor v. Rinderlunge (193 U/mg)/5 mg/l Bacitracin suspendiert. Nach dem Verdünnen auf 300 ml mit eiskaltem destilliertem Wasser wurde mit einem Ultraturrax (18-Stab, IKA Werk Staufen, Deutschland) 3 Minuten bei 75 % max. Leistung unter Eiskühlung homogenisiert. Nach Zugabe von 3 ml 1 M MgCl₂-Lösung (Endkonzentration 10 mM) ließ man exakt 1 Minute bei 0°C stehen. Durch Zugabe von Mg²⁺ aggregieren die Zellmembranen und präzipitieren mit Ausnahme der Bürstensaummembranen. Nach einer 15-minütigen Zentrifugation bei 3000 x g (5000 rpm, SS-34-Rotor) wurde der Niederschlag verworfen und der Überstand, der die Bürstensaummembranen enthielt, 30 Minuten bei 48000 x g (20000 rpm, SS-34-Rotor) zentrifugiert. Der Überstand wurde verworfen, der Niederschlag in 60 ml 12 mM Tris/HCl-Puffer (pH 7,1)/60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M MgCl₂-Lösung und 15-minütiger Inkubationszeit bei 0°C wurde erneut 15 Minuten bei 3000 x g zentrifugiert. Der Überstand wurde anschließend nochmals 30 Minuten bei 48000 x g (20000 rpm, SS-34-Rotor) zentrifugiert. Der Niederschlag wurde in 30 ml 10 mM Tris/Hepes-Puffer (pH 7,4)/300 mM Mannit aufgenommen und durch 20 Hübe in einem Potter Elvejhem Homogenisator bei 1000 rpm homogen resuspendiert. Nach 30 minütiger Zentrifugation bei 48000 x g (20000 rpm, SS-34-Rotor) wurde der Niederschlag in 0,5 bis 2 ml Tris/Hepes-Puffer (pH 7,4)/280 mM Mannit (Endkonzentration 20 mg/ml) aufgenommen und mit Hilfe einer Tuberkulinspritze mit einer 27 Gauge-Nadel resuspendiert. Die Vesikel wurden entweder unmittelbar nach der Präparation für Transportuntersuchungen verwendet oder bei -196°C in 4 mg Portionen in flüssigem Stickstoff aufbewahrt.

### 2. Hemmung der Na⁺-abhängigen [³H]Taurocholat-Aufnahme in Bürstensaummembranvesikel des Ileums

Die Aufnahme von Substraten in die vorstehend beschriebenen Bürstensaummembranvesikel wurde mittels der sogenannten Membranfiltrationstechnik bestimmt. 10 µl der Vesikelsuspension (100 µg Protein) wurden als Tropfen an die Wand eines Polystyrolinkubationsröhrchens (11 x 70 mm) pipettiert, welches das Inkubationsmedium mit den entsprechenden Liganden enthielt (90 µl). Das Inkubationsmedium enthielt 0,75 µl = 0,75 µCi [³H(G)]Taurocholat (spezifische Aktivität: 2,1 Ci/mMol)/0,5 µl 10 mM Taurocholat/8,75 µl Natrium-Transport-Puffer (10 mM Tris/Hepes (pH 7,4)/100 mM Mannit/100 mM NaCl) (Na-T-P) bzw. 8,75 µl Kalium-Transport-Puffer (10 mM Tris/Hepes (pH 7,4)/100 mM Mannit/100 mM KCI) (K-T-P) und 80 µl der betreffenden inhibitorlösung, je nach Experiment in Na-T-Puffer bzw. K-T-Puffer gelöst. Das Inkubationsmedium wurde durch ein Polyvinylidenfluorid-Membranfilter (SYHV LO 4NS, 0,45 µm, 4 mm ∅, Millipore, Eschborn, Deutschland) filtriert. Durch Vermischung der Vesikel mit dem Inkubationsmedium wurde die Transportmessung gestartet. Die Konzentration an Taurocholat im Inkubationsansatz betrug 50 µM. Nach der gewünschten Inkubationszeit (üblicherweise 1 Minute) wurde der Transport durch Zugabe von 1 ml eiskalter Stoplösung (10 mM Tris/Hepes (pH 7,4)/150 mM KCI) gestoppt. Die entstehende Mischung wurde sofort bei einem Vakuum von 25 bis 35 mbar über ein Membranfilter aus Cellulosenitrat (ME 25, 0,45 µm, 25 mm Durchmesser, Schleicher & Schuell, Dassell, Deutschland) abgesaugt. Der Filter wurde mit 5 ml eiskalter Stoplösung nachgewaschen.

Zur Messung der Aufnahme des radioaktiv markierten Taurocholats wurde das Membranfilter mit 4 ml des Szintillators Quickszint 361 (Zinsser Analytik GmbH, Frankfurt, Deutschland) aufgelöst und die Radioaktivität durch Flüssigkeits-Szintillationsmessung in einem Meßgerät TriCarb 2500 (Canberra Packard GmbH, Frankfurt, Deutschland) gemessen. Die gemessenen Werte wurden nach Eichung des Gerätes mit Hilfe von Standardproben und nach Korrektur evtl. vorhandener Chemilumineszenz als dpm (Decompositions per Minute) erhalten. Die Kontrollwerte wurden jeweils in Na-T-P und K-T-P ermittelt. Die Differenz zwischen der Aufnahme in Na-T-P und K-T-P ergab den Na⁺-abhängigen Transportanteil. Als IC₅₀ Na⁺ wurde diejenige Konzentration an Inhibitor bezeichnet, bei der der Na⁺-abhängige Transportanteil um 50 % - bezogen auf die Kontrolle-gehemmt war.

Die pharmakologischen Daten umfassen eine Testserie, in der die Interaktion der erfindungsgemäßen Verbindungen mit dem intestinalen Gallensäuretransportsystem im terminalen Dünndarm untersucht wurde. Die Ergebnisse sind in Tabelle 1 zusammengefaßt.

Tabelle 1 zeigt Meßwerte der Hemmung der [³H]-Taurocholataufnahme in Bürsensaummembranvesikel des Ileums von Kaninchen. Angegeben sind die Quotienten aus den IC_{50Na}-Werten der Referenzsubstanz als Taurochenodesoxycholat (TCDC) und der jeweiligen Testsubstanz.

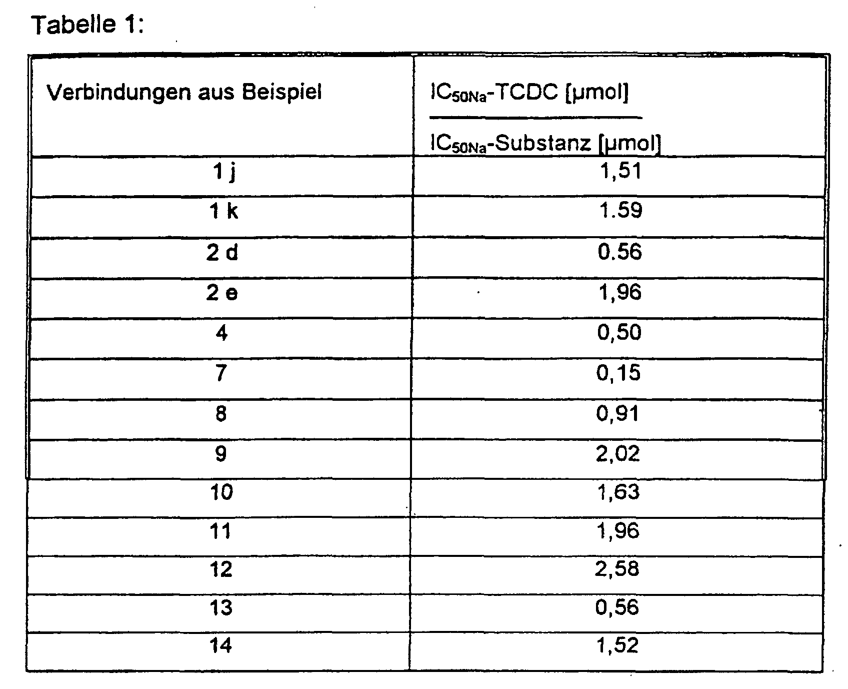

Die nachfolgenden Beispiele dienen zur näheren Erläuterung der Erfindung, ohne dieselbe auf in den Beispielen beschriebene Produkte und Ausführungsformen einzuschränken.

### Beispiel 1

### a.

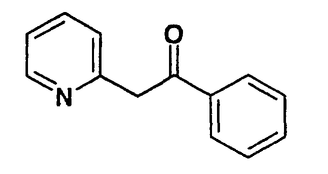

Zu 50 g (0.54 mol) Picolin in 770 ml Tetrahydrofuran wurden bei -55°C 366 ml 15 % n-Butyllithium in n-Hexan zugetropft. Es wurde auf Zimmertemperatur erwärmt und erneut auf -55°C gekühlt. 77 g N,N-Dimethylbenzamid (0.52 mol) in 570 ml Tetrahydrofuran wurden langsam zugetropft, anschließend auf Zimmertemperatur erwärmt und noch 1 h gerührt. Nach der Zugabe von 550 ml 1 N Salzsäure wurde mit Ethylacetat extrahiert (3x), die organischen Phasen mit MgSO₄ getrocknet und eingedampft. Durch Destillation des Rückstandes wurden 47.5 g (47%) Produkt erhalten. Siedepunkt 134-136°C/0.28 mbar.

### b.

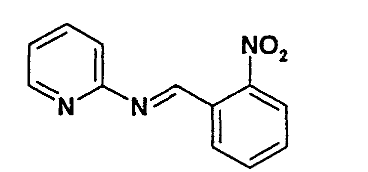

20.0 g (0.13 mol) o-Nitrobenzaldehyd, 12.5 g (0.13 mol) 2-Aminopyridin und 0.3 g p-Toluolsulfonsäure wurden in 150 ml Toluol 2.5 h am Wasserabscheider unter Rückfluß erhitzt. Die Lösung wurde abgekühlt, der entstandene Niederschlag abgesaugt und getrocknet.
Ausbeute: 18.1 g (60%) Produkt
Schmelzpunkt: 93-95°C
C₁₂H₉N₃O₂ (227) MS (FAB) 228 M + H⁺

### C.

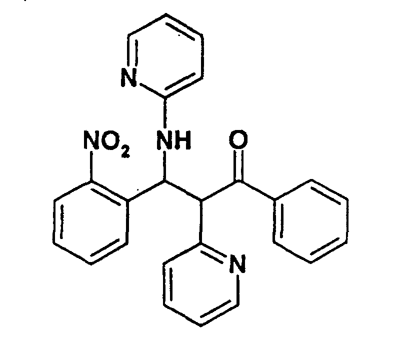

12.0 g (61 mmol) Keton aus Beispiel 1 a und 15.0 g (66 mmol) Imin aus Beispiel 1 b wurden 45 min auf dem Dampfbad erwärmt. Das Reaktionsgemisch wurde in Ethanol unter Erwärmen gelöst. Nach dem Abkühlen wurde der Niederschlag abgesaugt und aus Ethanol umkristallisiert.
Ausbeute: 11.8 g (46%) Produkt
C₂₅H₂₀N₄O₃ (424.2) MS (FAB) 425 M + H⁺

### d.

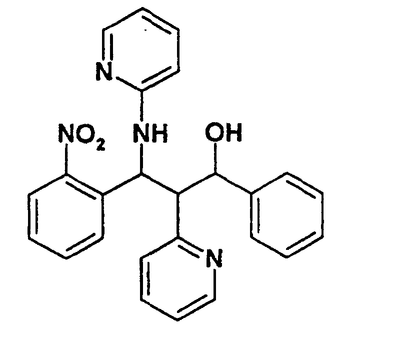

8.0 g (18.8 mmol) Ketoverbindung aus Beispiel 1 c wurden in 300 ml Tetrahydrofuran/Wasser 10:1 gelöst, mit 4.67 g Natriumborhydrid versetzt und 2 h bei Zimmertemperatur gerührt. Anschließend wurde die Lösung eingedampft, der Rückstand mit 100 ml 2N Salzsäure versetzt und auf dem Dampfbad erwärmt bis alles gelöst war. Nach dem Abkühlen wurde mit 4N NaOH-Lösung basisch gestellt und mit Ethylacetat (2x) extrahiert. Die organischen Phasen wurden mit MgSO₄ getrocknet und eingedampft. Der Rückstand wurde über Kieselgel chromatographiert (Heptan/Ethylacetat 1:1). Es wurden zwei racemische Verbindungen als Produkt erhalten.
1. Fraktion: 3.9 g (48 %) unpolares Racemat (Bsp. 1 d/1)
   C₂₅H₂₂N₄O₃ (426.2) MS (FAB) 427 M + H⁺
2. Fraktion: 2.5 g (31 %) polares Racemat (Bsp. 1 d/2)
   C₂₅H₂₂N₄O₃ (426.2) MS (FAB) 427 M + H⁺

### e.

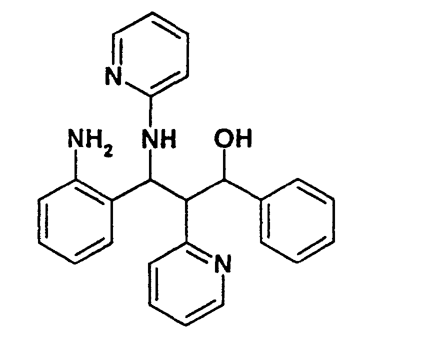

2.5 g (5.86 mmol) des unpolaren Racemats aus Beispiel 1 d/1 wurden in 300 ml Methanol gelöst, mit ca. 20 mg Pd/C 10 % versetzt und unter H₂-Atmosphäre bei Zimmertemperatur hydriert. Vom Katalysator wurde abfiltriert und die Lösung eingedampft. Der Rückstand wurde über Kieselgel chromatographiert (n-Heptan/ Ethylacetat 7:13).
Ausbeute: 1.9 g (82%) Produkt
C₂₅H₂₄N₄O (396.22) MS (FAB) 397 M + H⁺

### f.

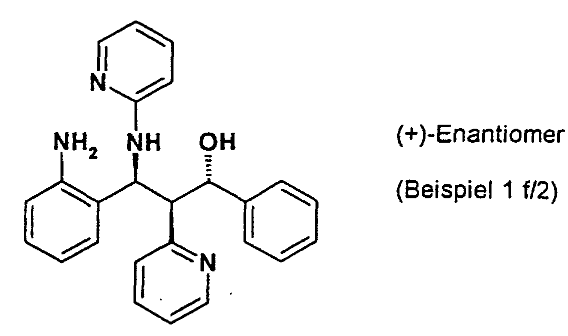

100 mg der racematischen Verbindung aus Beispiel 1 e wurde durch präparative HPLC in die Enantiomere getrennt. Die Trennung erfolgte über eine CSP-Chiralpak-Säule (Fa. Daicel, Düsseldorf) mit n-Hexan/Ethanol 4:1.
Als erste Fraktion wurden 40 mg des (-)-Enantiomers (Bsp. 1 f/1) als 2. Fraktion 40 mg des (+)-Enantiomers (Bsp. 1 f/2) erhalten.

### g.

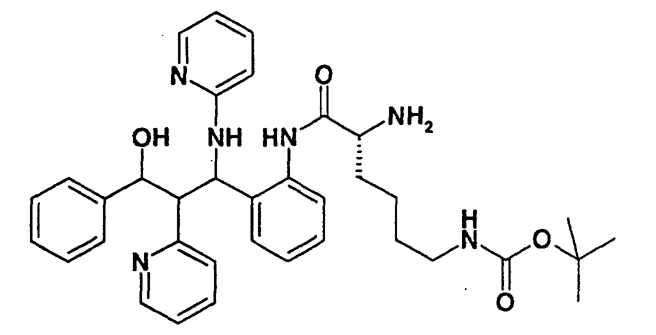

4.0 g (10.1 mmol) der Aminoverbindung aus Beispiel 1e (unpolares Racemat), 4.85 g (10.3 mmol) N-Fmoc-D-Lys(BOC)OH, 4.0 g (12.2 mmol) TOTU und 2.7 ml Triethylamin wurden in 300 ml Dimethylformamid gelöst und 2 h bei Zimmertemperatur gerührt. Das Reaktionsgemisch wurde auf Wasser gegossen und mit Ethylacetat (2 x) extrahiert. Die organischen Phasen wurden getrocknet (MgSO₄) und eingedampft. Der Rückstand wurde in 150 ml Dimethylformamid/Piperidin 2:1 zur Abspaltung der Fmoc-Gruppe gelöst und 1 h bei Zimmertemperatur gerührt. Es wurde auf Wasser gegossen, mit Ethylacetat (3 x) extrahiert. Die organischen Phasen wurden getrocknet (MgSO₄) und eingedampft. Chromatographie über Kieselgel (Dichlormethan/Methanol 9:1) ergab 4.0 g (63.5 %) Produkt.
C₃₆H₄₄N₆O₄ (624.3) MS (FAB) 625 M + H⁺

### h.

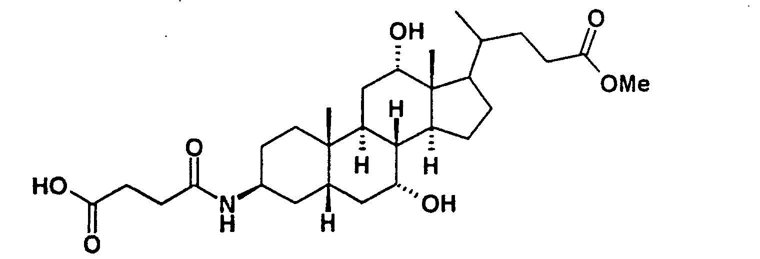

5.0 g (11.86 mmol) 3β-Aminocholsäuremethylester (Europäische Patentanmeldung EP 0614908), 1,3 g (13 mmol) Bernsteinsäureanhydrid und 16.5 ml Triethylamin wurden in 75 ml Tetrahydrofuran gelöst und 1 h bei Zimmertemperatur gerührt. Die Lösung wurde eingedampft. Der Rückstand wurde in Wasser gelöst, mit Salzsäure angesäuert, mit Ethylacetat extrahiert (3 x). Die organischen Phasen wurden getrocknet (MgSO₄) und eingedampft.
Ausbeute: 5,8 g (94 %)
C₂₉H₄₇NO₇ (521.3) MS (FAB) 528 M + LI⁺

### i.

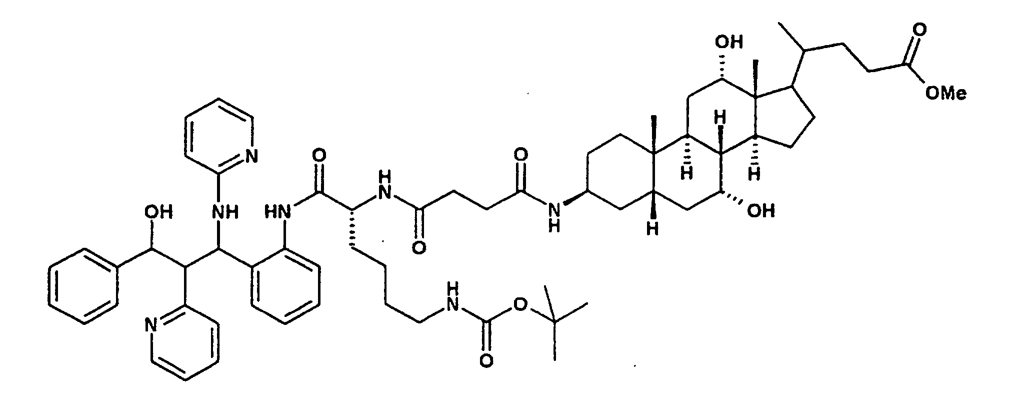

4.0 g (6.4 mmol) der Verbindung aus Beispiel 1 g, 3.45 g (6.6 mmol) Gallensäurederivat aus Beispiel 1 h, 1.2 ml Triethylamin, 2.16 g (16 mmol) Hydroxybenzotriazol und 2.56 g Dicyclohexylcarbodimid (12.4 mmol) wurden in 250 ml Tetrahydrofuran gelöst und 5 h bei Zimmertemperatur gerührt. Das Gemisch wurde eingedampft, der Rückstand in Ethylacetat gelöst und mit NaHCO₃-Lösung gewaschen. Die organischen Phasen wurden getrocknet (MgSO₄) und eingedampft. Chromatographie über Kieselgel (Dichlormethan/Methanol 19:1, dann 9:1) ergab 3.1 g (43 %) Produkt.
C₆₅H₈₉N₇O₁₀ (1127.7) MS (FAB) 1134.7 M + Li⁺

### j.

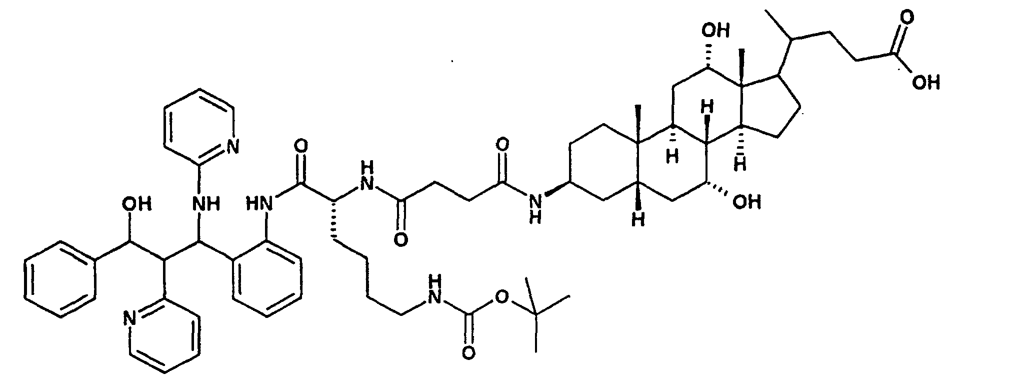

3.1 g (2.75 mmol) Methylester aus Beispiel 1 i wurden in 200 ml Ethanol gelöst, mit 31 ml 1N NaOH-Lösung versetzt und 5 h bei Zimmertemperatur gerührt. Das Gemisch wurde eingedampft, der Rückstand in Wasser gelöst und mit gesättigter NaH₂PO₄-Lösung versetzt. Es wurde mit Ethylacetat extrahiert (2 x), die organischen Phasen über MgSO₄ getrocknet und eingedampft. Das Rohprodukt wurde über Kieselgel chromatographiert (Dichlormethan/Methanol 4:1).
Ausbeute: 2.25 g (73 %)
C₆₄H₈₇N₇O₁₀ (1113.7) MS (FAB) 1120.7 M + Li⁺

### k.

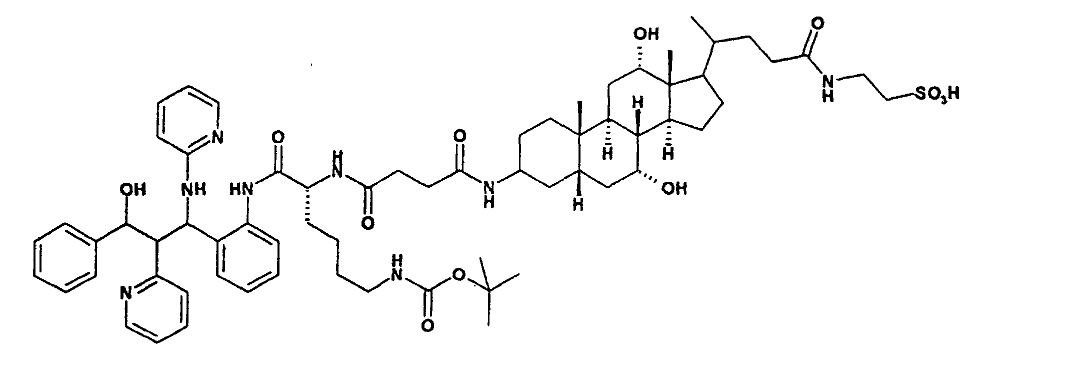

1.5 g (1.35 mmol) der Verbindung aus Beispiel 1 j und 0.81 ml Triethylamin wurden bei 0° C mit 0.48 ml Chlorameisensäureethylester versetzt und 10 min gerührt. Danach wurden 0.6 g Taurin, gelöst in 30 ml 0.1 N NaOH-Lösung, zugegeben und 24 h bei Zimmertemperatur gerührt. Das Gemisch wurde eingedampft, der Rückstand in wenig Wasser gelöst und zu gesättigter NaH₂PO₄-Lösung gegossen. Es wurde mit Ethylacetat extrahiert (3 x), die organischen Phasen mit MgSO₄ getrocknet und eingedampft. Nach Chromatographie über Kieselgel (Dichlormethan/Methanol 4:1, dann Methanol) wurden 0.98 g (60 %) Taurinkonjugat erhalten.
C₆₆H₉₂N₈O₁₂S (1270.7) MS (FAB) 1243.6 M + Na⁺

### Beispiel 2

### a.

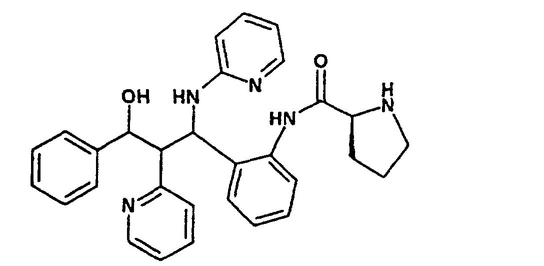

2.5 g (6.31 mmol) Aminoverbindung aus Beispiel 1 e (unpolares Racemat), 2.2 g (6.52 mmol) Fmoc-L-Prolin, 2.5 g (7.62 mmol) TOTU und 1.7 ml Triethylamin wurden in 100 ml Dimethylformamid gelöst und 3 h bei Zimmertemperatur gerührt. Das Reaktionsgemisch wurde auf die Hälfte eingedampft, mit Wasser versetzt und mit Ethylacetat extrahiert (3 x). Die organischen Phasen wurden über MgSO₄ getrocknet und eingedampft. Nach Chromatographie über Kieselgel (Ethylacetat/n-Heptan 7:3) wurden 3.85 g (85 %) Produkt erhalten.

Dieses Fmoc-geschützte Zwischenprodukt (3.6 g) wurde in 110 ml Piperidin/DMF 1:10 gelöst und 1 h bei Zimmertemperatur gerührt. Das Gemisch wurde eingedampft und über Kieselgel (Dichlormethan/Methanol 19:1), dann 9: 1) chromatographiert.
Ausbeute: 1.8 g (72.5 %)
C₃₀H₃₁N₅O₂ (493.2) MS (FAB) 494 M + H⁺

### b.

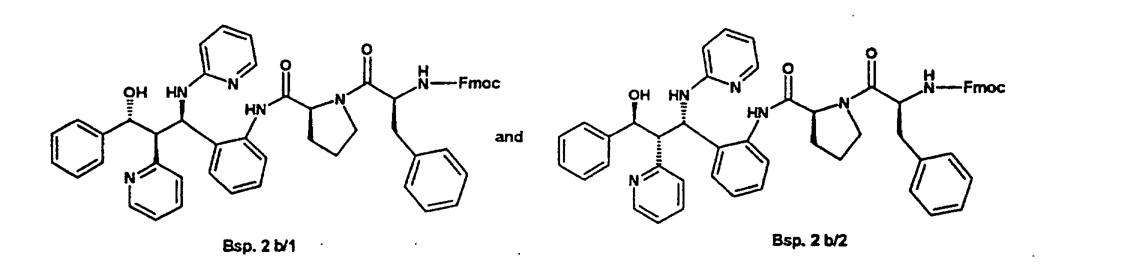

1.7 g (3.44 mmol) der Verbindung aus Beispiel 2 a wurden mit 1.4 g (3.61 mmol) Fmoc-L-Phenylalanin, 1.9 g (5.80 mmol) TOTU und 1.0 ml Triethylamin in 150 ml DMF 4 h bei Zimmertemperatur gerührt. Das Reaktionsgemisch wurde eingedampft und der Rückstand über Kieselgel chromatographiert (Ethylacetat/n-Heptan 4:1). Es wurden zwei Fraktionen erhalten:
1. Fraktion 1.28 g (43 %) unpolares Diastereomer (Bsp. 2 b/1)
   C₅₄H₅₀N₆O₅ (862.4) MS (FAB) 863.4 M + H⁺
2. Fraktion 0.82 g (28 %) polares Diastereomer (Bsp. 2 b/1)
   C₅₄H₅₀N₆O₅ (862.4) MS(FAB) 863.4 M + H⁺

### c.

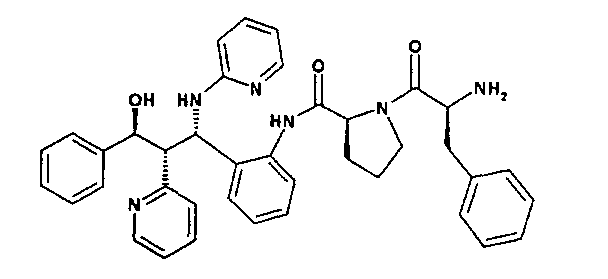

0.8 g (0.93 mmol) Verbindung aus Beispiel 2 b/2 wurden in 33 ml DMF/Piperidin 10:1 gelöst und 1 h bei Zimmertemperatur gerührt. Nach dem Eindampfen wurde der Rückstand über Kieselgel chromatographiert (Dichlormethan/Methanol 19:1, dann 9:1).
Ausbeute: 0.35 g (59 %).
C₃₉H₄₀N₆O₃(640.3) MS (FAB) 641.3 M + H⁺

### d.

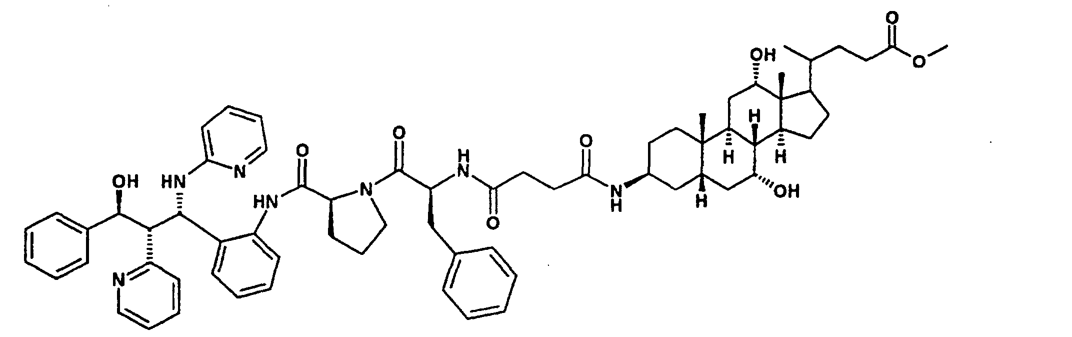

0.5 g (0.78 mmol) der Verbindung aus Beispiel 2 c, 0.45 g (0.86 mmol) des Gallensäurederivats aus Beispiel 1 h wurden nach dem für Beispiel 1 i beschriebenen Verfahren zur Reaktion gebracht. Es wurden 0.38 g (43 %) Produkt erhalten.
C₆₈H₈₅N₇O₉ (1143.6) MS (FAB) 1144.6 M + H⁺

### e.

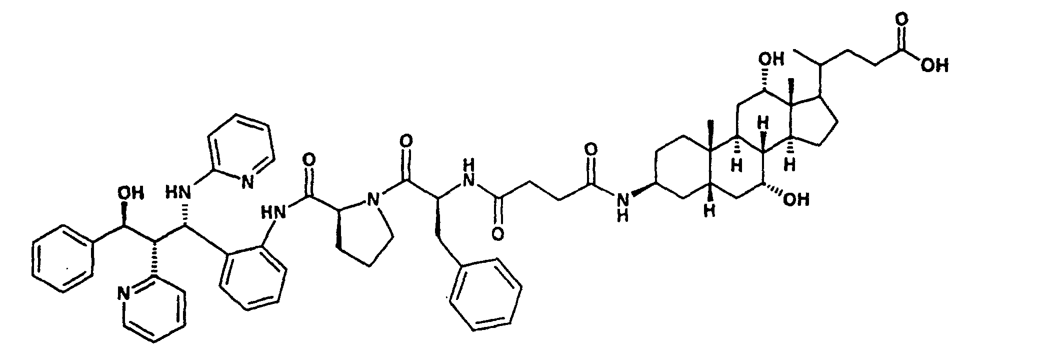

0.31 g (0.27 mmol) des Methylesters aus Beispiel 1 d wurden in 30 ml Ethanol gelöst, mit 3.0 ml 1 N NaOH-Lösung versetzt und 12 h bei Zimmertemperatur gerührt. Das Reaktionsgemisch wurde eingedampft und der Rückstand über Kieselgel chromatographiert (Dichlormethan/Methanol 4:1).
Ausbeute: 220 mg (72 %)
C₆₇H₈₃N₇O₉ (1129.6) MS (FAB) 1130.6 M + H⁺

### Beispiel 3

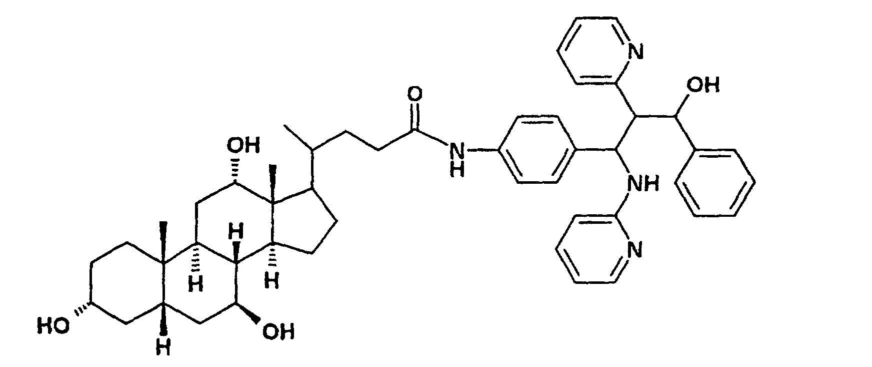

0.3 g (0.78 mmol) 3-(4-Aminophenyl)-1-phenyl-2-pyridin-2-yl-3-(pyridin-2-ylamino)-propan-1-ol (Herstellung analog zu Beispiel 1 e), 0.34 g (0.83 mmol) Ursocholsäure, 0.34 g (2.52 mmol) Hydroxybenzotriazol, 0.41 g (2 mmol) Dicyclohexylcarbodiimid und 0.15 ml Triethylamin wurden in 50 ml Tetrahydrofuran 2 Tage bei Zimmertemperatur gerührt. Nach Beendigung der Reaktion wurden die Feststoffe abfiltriert. Die Lösung wurde eingedampft und der Rückstand über Kieselgel chromatographiert (Dichlormethan/Methanol 9:1, dann 17:3). Es wurden 0.33 g (55 %) Produkt erhalten.
C ₄₉H₆₂N₄O₅ (786.5) MS (FAB) 787.5 M + H⁺

Ausgehend von den entsprechenden Ausgangsverbindungen wurden in Analogie zu den Beispielen 1 bis 3 die Beispiele 4 bis 14 erhalten.

### Beispiel 4

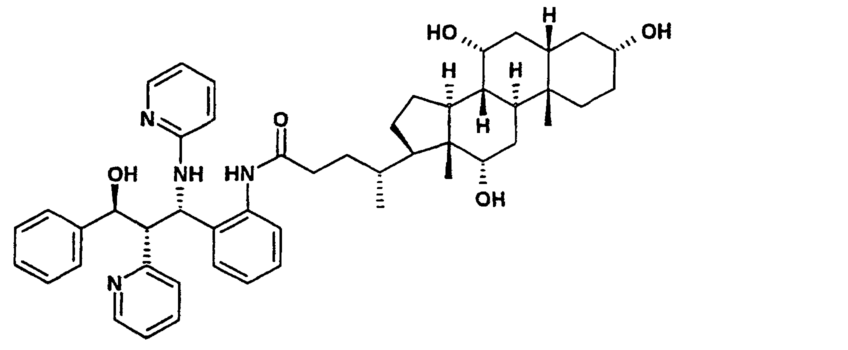

C₄₉H₆₂N₄O₅ (787,1) MS (FAB) 788,1 M+H⁺

### Beispiel 5 (unpolares Diastereomer)

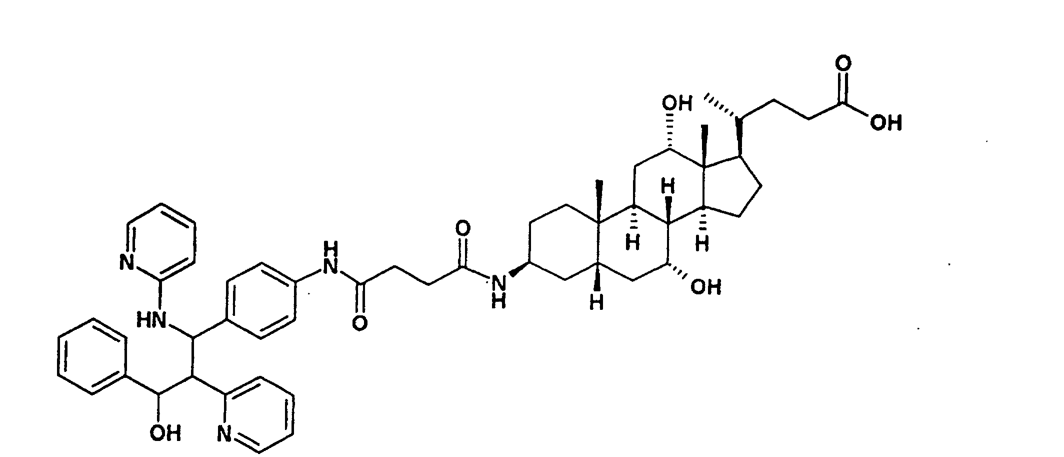

C₅₃H₆₇N₅O₇ (886,2) MS (FAB) 887,2 M+H⁺

### Beispiel 6

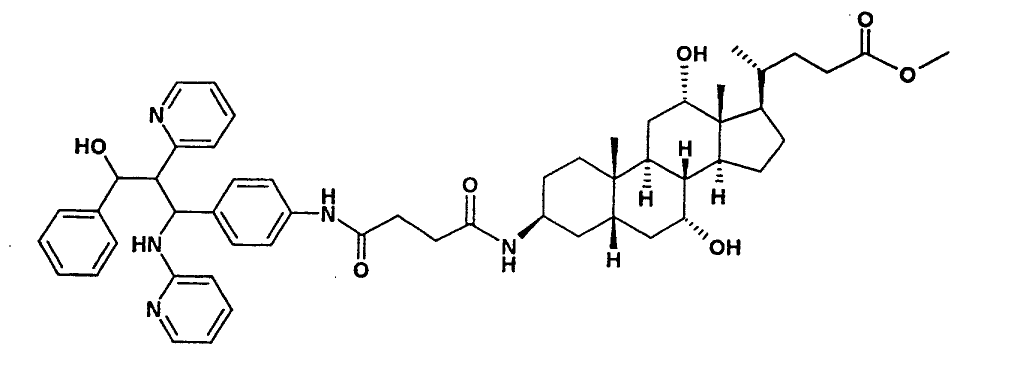

C₅₄H₆₉N₅O₇ (900,2) MS (FAB) 901,2 M+H⁺

### Beispiel 7 (polares Diastereomer)

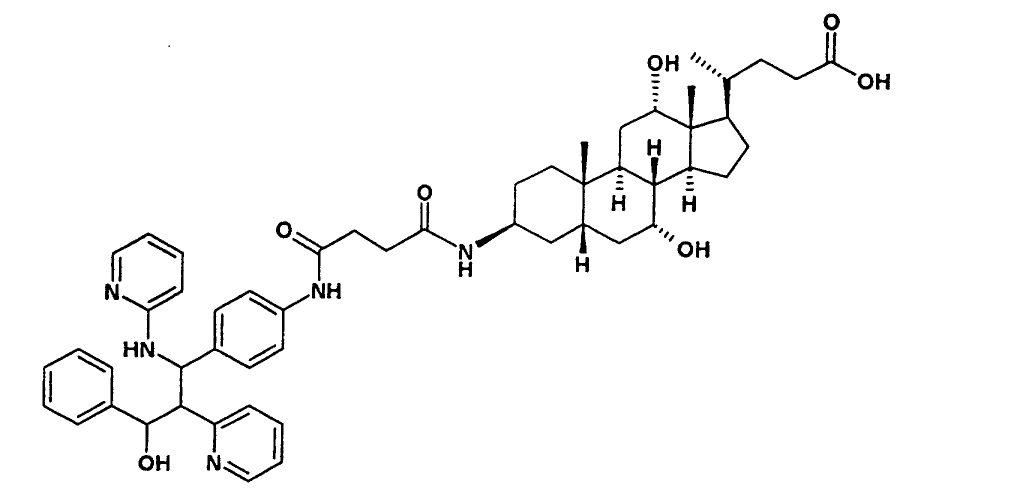

C₅₃H₆₇N₅O₇ (886,2) MS (FAB) 887,2 M+H⁺

### Beispiel 8

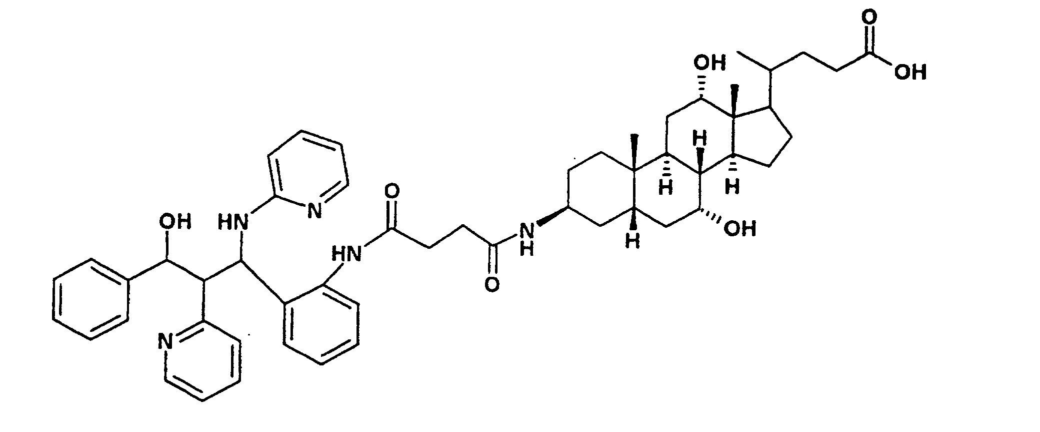

C₅₃H₆₇N₅O₇ (886,2) MS (FAB) 887,2 M+H⁺

### Beispiel 9

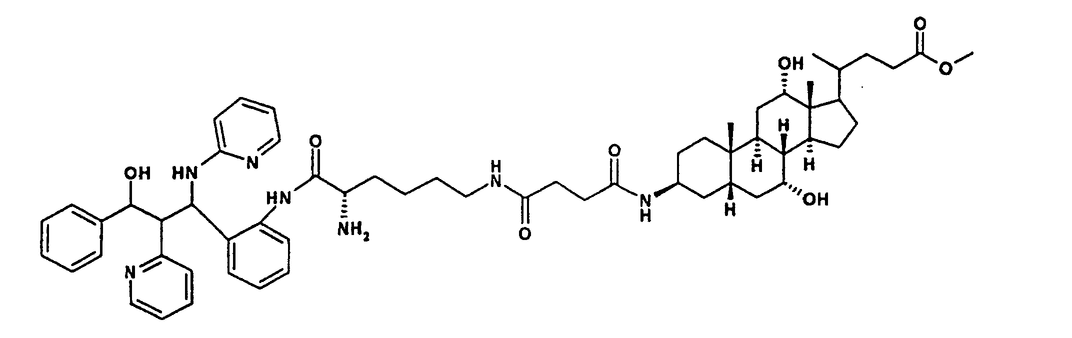

C₆₀H₈₁N₇O₈ (1028,4) MS (FAB) 1029,4 M+H⁺

### Beispiel 10

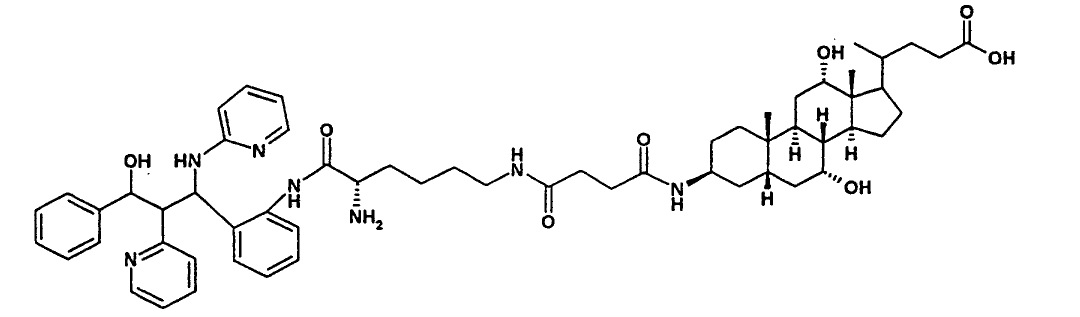

C₅₉H₇₉N₇O₈ (1014,3) MS (FAB) 1015,3 M+H⁺

### Beispiel 11

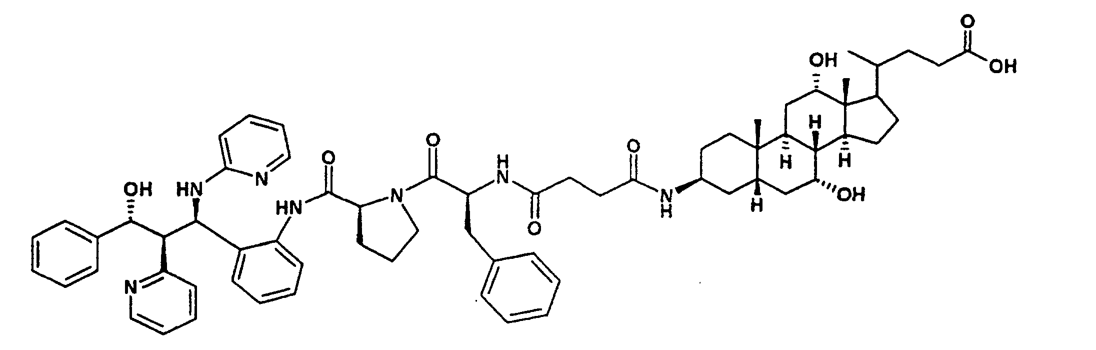

C₆₇H₈₃N₇O₉ (1130,5) MS (FAB) 1031,5 M+H⁺

### Beispiel 12

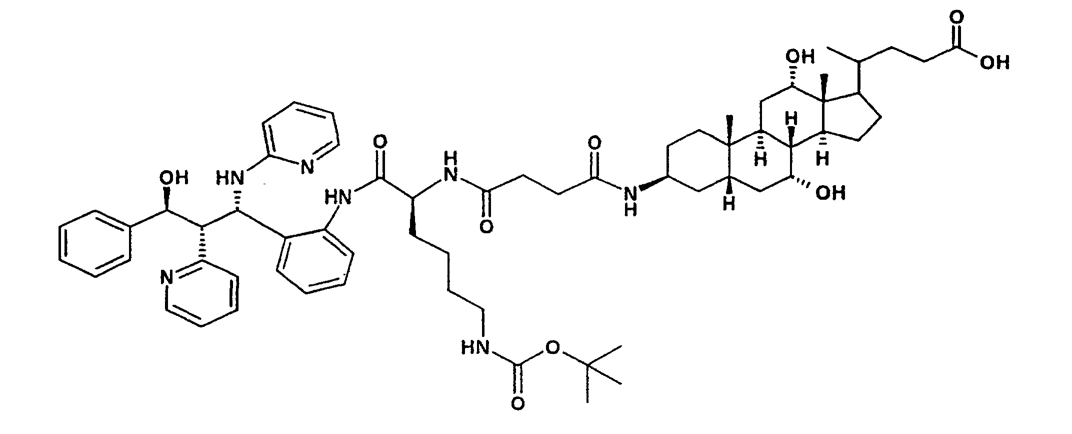

C₆₄H₈₇N₇O₁₀ (1114,4) MS (FAB) 1115,4 M+H⁺

### Beispiel 13

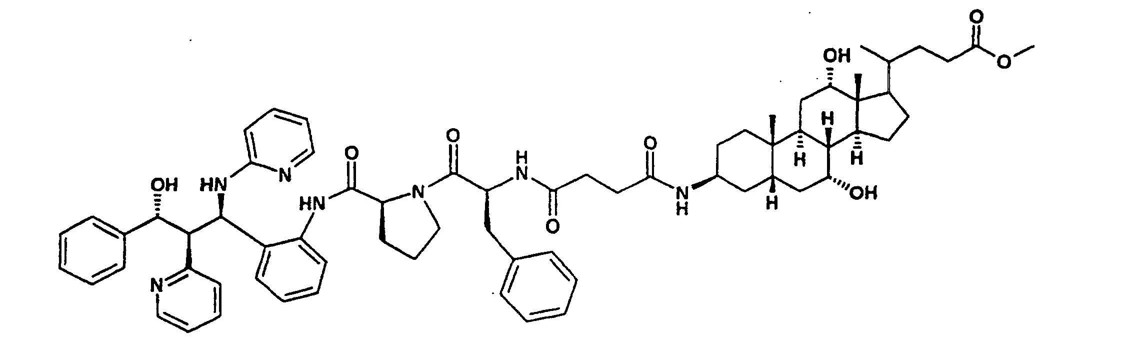

C₆₈H₈₅N₇O₉ (1144,5) MS (FAB) 1145,5 M+H⁺

### Beispiel 14

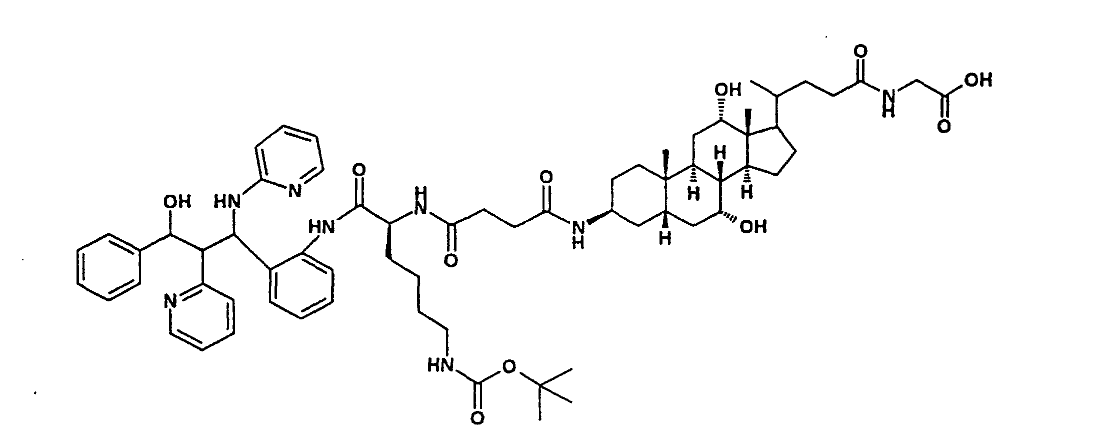

C₆₆H₉₀N₈O₁₁ (1171,5) MS (FAB) 1172,5 M+H⁺

## Patentansprüche

1. Verbindungen der Formel I, 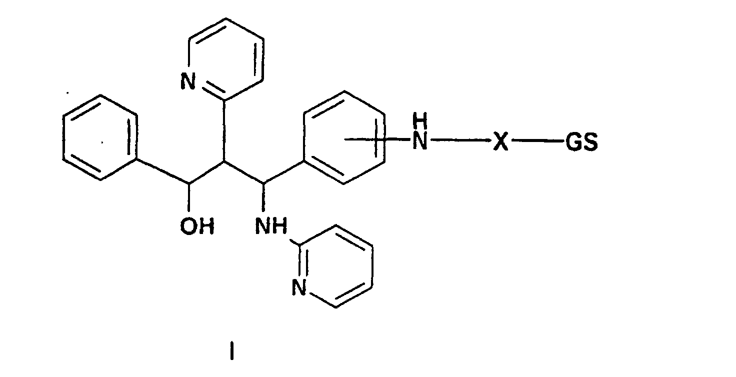 worin bedeuten
GS eine Gallensäuregruppe der Formel 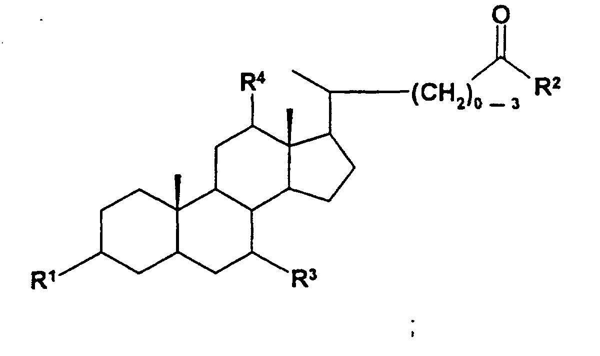
R¹ eine Bindung zu X, OH;
R² eine Bindung zu X, OH, O-(C₁-C₆)-Alkyl, NH-(C₂-C₆)-Alkyl-SO₃H, N(CH₃)-CH₂-CH₂-SO₃H, NH-(C₁-C₆)-Alkyl-COOH; N(CH₃)-(C₁-C₆)-Alkyl-COOH;
mit der Maßgabe, daß R¹ und R² nicht gleichzeitig die folgende Bedeutung haben
R¹ eine Bindung zu X und
R² eine Bindung zu X;
R³ , R⁴ unabhängig voneinander H, OH;
X 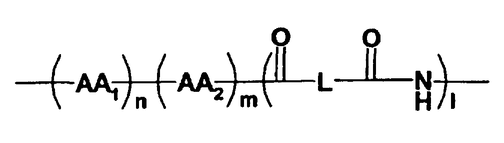 oder eine Bindung;
l, m, n unabhängig voneinander 0 oder 1;
L (C₁ - C₆)-Alkyl, Phenyl;
AA₁, AA₂ unabhängig voneinander einen Aminosäurerest oder einen Aminosäurerest, der einfach oder mehrfach substituiert ist durch eine Aminosäureschutzgruppe;
sowie deren pharmazeutisch verträgliche Salze.

2. Verbindungen der Formel I, gemäß Anspruch 1, **dadurch gekennzeichnet, daß** darin bedeuten
GS eine Gallensäuregruppe der Formel 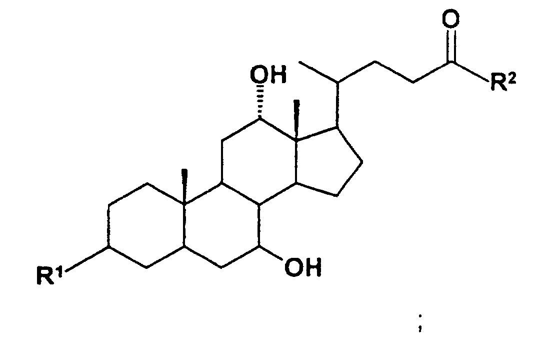
R¹ eine Bindung zu X, OH;
R² eine Bindung zu X, OH, O-(C₁-C₆)-Alkyl , NH-(C₂-C₆)-Alkyl-SO₃H, N(CH₃)-CH₂-CH₂-SO₃H, NH-(C₁-C₆)-Alkyl-COOH; N(CH₃)-(C₁-C₆)-Alkyl-COOH;
mit der Maßgabe, daß R¹ und R² nicht gleichzeitig die folgende Bedeutung haben
R¹ eine Bindung zu X und
R² eine Bindung zu X;
X 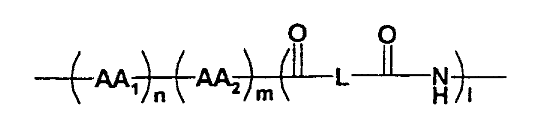 oder eine Bindung;
l, m, n unabhängig voneinander 0 oder 1;
L (C₁ - C₆)-Alkyl, Phenyl;
AA₁ , AA₂ unabhängig voneinander einen Aminosäurerest oder einen Aminosäurerest, der einfach oder mehrfach substituiert ist durch eine Aminosäureschutzgruppe;
sowie deren pharmazeutisch verträgliche Salze und physiologisch.

3. Verbindungen der Formel I, gemäß Anspruch 1 oder 2, **dadurch gekennzeichnet, daß** darin bedeuten
GS eine Gallensäuregruppe der Formel 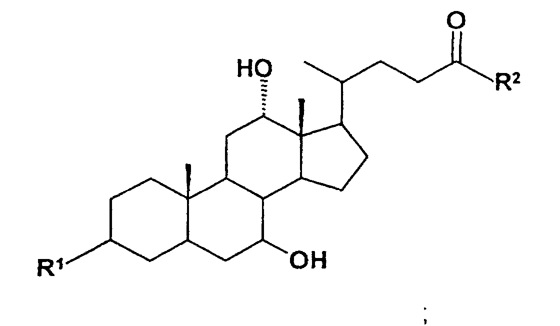
R¹ eine Bindung zu X, OH;
R² eine Bindung zu X, OH, O-(C₁-C₆)-Alkyl, NH-(C₂-C₆)-Alkyl-SO₃H, NH-(C₁-C₆)-Alkyl-COOH;
mit der Maßgabe, daß R¹ und R² nicht gleichzeitig die folgende Bedeutung haben
R¹ eine Bindung zu X und
R² eine Bindung zu X;
X 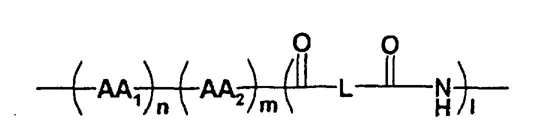 oder eine Bindung;
l, m, n unabhängig voneinander 0 oder 1;
L (C₁ - C₆)-Alkyl;
AA₁ , AA₂ unabhängig voneinander einen Aminosäurerest oder einen Aminosäurerest, der einfach oder mehrfach substituiert ist durch eine Aminosäureschutzgruppe;
sowie deren pharmazeutisch verträgliche Salze.

4. Arzneimittel enthaltend eine oder mehrere der Verbindungen gemäß einem oder mehreren der Ansprüche 1 bis 3.

5. Arzneimittel enthaltend eine oder mehrere der Verbindungen gemäß einem oder mehreren der Ansprüche 1 bis 3 und ein oder mehrere lipidsenkende Wirkstoffe.

6. Verbindungen gemäß einem oder mehreren der Ansprüche 1 bis 3 zur Anwendung als Medikament zur Prophylaxe oder Behandlung von Lipidstoffwechselstörungen.

7. Verbindungen gemäß einem oder mehreren der Ansprüche 1 bis 3 zur Anwendung als Medikament zur Behandlung von Hyperlipidämie.

8. Verbindungen gemäß einem oder mehreren der Ansprüche 1 bis 3 zur Anwendung als Medikament zur Prophylaxe oder Behandlung von arteriosklerotischer Erscheinungen.

9. Verbindungen gemäß einem oder mehreren der Ansprüche 1 bis 3 in Kombination mit mindestens einem weiteren lipidsenkenden Wirkstoff zur Anwendung als Medikament zur Prophylaxe oder Behandlung von Lipidstoffwechselstörungen.

10. Verbindungen gemäß einem oder mehreren der Ansprüche 1 bis 3 in Kombination mit mindestens einem weiteren lipidsenkenden Wirkstoff als Medikament zur Behandlung von Hyperlipidämie.

11. Verbindungen gemäß einem oder mehreren der Ansprüche 1 bis 3 in Kombination mit mindestens einem weiteren lipidsenkenden Wirkstoff zur Anwendung als Medikament zur Prophylaxe oder Behandlung von arteriosklerotischer Erscheinungen.

12. Verfahren zur Herstellung eines Arzneimittels enthaltend eine oder mehrere der Verbindungen gemäß einem oder mehreren der Ansprüche 1 bis 3, **dadurch gekennzeichnet, daß** der Wirkstoff mit einem pharmazeutisch geeigneten Träger vermischt wird und diese Mischung in eine für die Verabreichung geeignete Form gebracht wird.

13. Verwendung der Verbindungen gemäß einem oder mehreren der Ansprüche 1 bis 3 zur Herstellung eines Medikaments zur Prophylaxe oder Behandlung von Lipidstoffwechselstörungen.

14. Verwendung der Verbindungen gemäß einem oder mehreren der Ansprüche 1 bis 3 zur Herstellung eines Medikaments zur Behandlung von Hyperlipidämie.

## Claims

1. A compound of the formula I 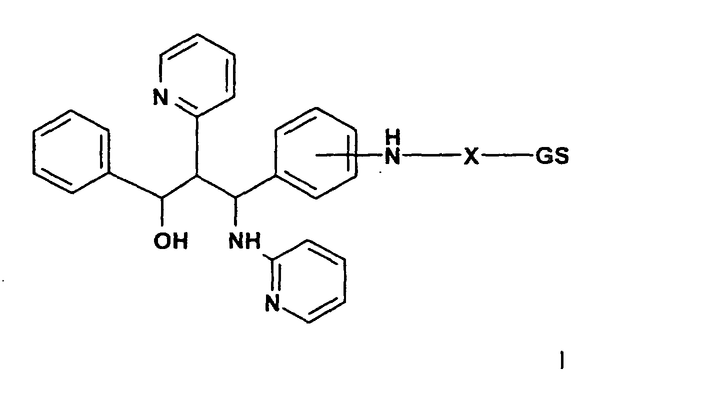 in which
GS is a bile acid group of the formula 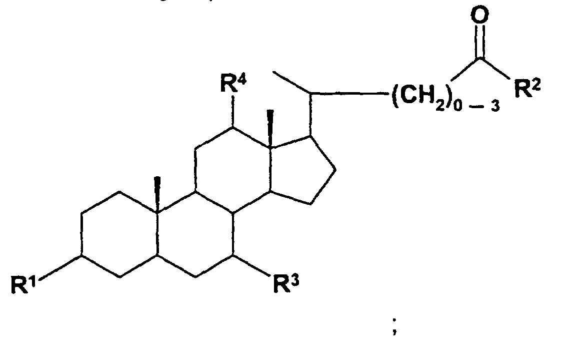
R¹ is a bond to X, OH;
R² is a bond to X, OH, O-(C₁-C₆)-alkyl, NH-(C₂-C₆)-alkyl-SO₃H, N(CH₃)-CH₂-CH₂-SO₃H, NH-(C₁-C₆)-alkyl-COOH; N(CH₃)-(C₁-C₆)-alkyl-COOH;
with the proviso that R¹ and R² do not simultaneously have the following meaning
R¹ a bond to X and
R² a bond to X;
R³, R⁴ independently of one another are H, OH;
X is 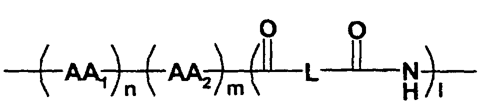 or a bond;
l, m, n independently of one another are 0 or 1;
L is (C₁ - C₆)-alkyl, phenyl;
AA₁, AA₂ independently of one another are an amino acid radical or an amino acid radical which is mono- or polysubstituted by an amino acid-protective group;
and pharmaceutically tolerated salts thereof.

2. A compound of the formula I as claimed in claim 1, in which
GS is a bile acid group of the formula 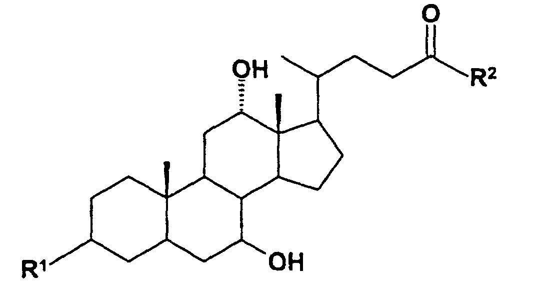
R¹ is a bond to X, OH;
R² is a bond to X, OH, O-(C₁-C₆)-alkyl, NH-(C₂-C₆)-alkyl-SO₃H, N(CH₃)-CH₂-CH₂-SO₃H, NH-(C₁-C₆)-alkyl-COOH; N(CH₃)-(C₁-C₆)-alkyl-COOH;
with the proviso that R¹ and R² do not simultaneously have the following meaning
R¹ a bond to X and
R² a bond to X;
X a 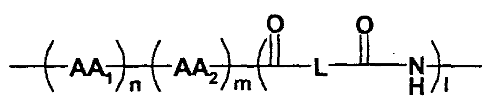 or a bond;
l, m, n independently of one another are 0 or 1;
L is (C₁ - C₆)-alkyl, phenyl;
AA₁, AA₂ independently of one another are an amino acid radical or an amino acid radical which is mono- or polysubstituted by an amino acid-protective group;
and pharmaceutically tolerated salts thereof.

3. A compound of the formula I as claimed in claim 1 or 2, in which
GS is a bile acid group of the formula 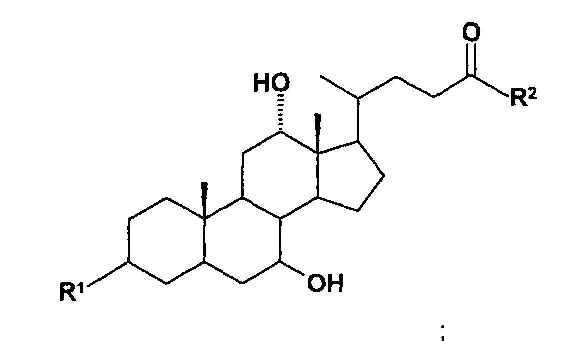
R¹ is a bond to X, OH;
R² is a bond to X, OH, O-(C₁-C₆)-alkyl, NH-(C₂-C₆)-alkyl-SO₃H, NH-(C₁-C₆)-alkyl-COOH;
with the proviso that R¹ and R² do not simultaneously have the following meaning
R¹ a bond to X and
R² a bond to X;
X is 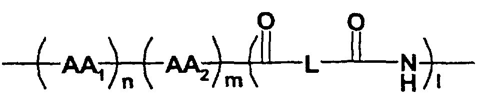 or a bond;
l, m, n independently of one another are 0 or 1;
L is (C₁ - C₆)-alkyl;
AA₁, AA₂ independently of one another are an amino acid radical or an amino acid radical which is mono- or polysubstituted by an amino acid-protective group;
and pharmaceutically tolerated salts thereof.

4. A pharmaceutical comprising one or more of the compounds as claimed in one or more of claims 1 to 3.

5. A pharmaceutical comprising one or more of the compounds as claimed in one or more of the claims 1 to 3 and one or more lipid-lowering active compounds.

6. A compound as claimed in one or more of claims 1 to 3 for use as a pharmaceutical for prophylaxis or treatment of disturbances in lipid metabolism.

7. A compound as claimed in one or more of claims 1 to 3 for use as a pharmaceutical for treatment of hyperlipidemia.

8. A compound as claimed in one or more of claims 1 to 3 for use as a pharmaceutical for prophylaxis or treatment of arteriosclerotic symptoms.

9. A compound as claimed in one or more of claims 1 to 3 in combination with at least one further lipid-lowering active compound for use as a pharmaceutical for prophylaxis or treatment of disturbances in lipid metabolism.

10. A compound as claimed in one or more of claims 1 to 3 in combination with at least one further lipid-lowering active compound as a pharmaceutical for treatment of hyperlipidemia.

11. A compound as claimed in one or more of claims 1 to 3 in combination with at least one further lipid-lowering active compound for use as a pharmaceutical for prophylaxis or treatment of arteriosclerotic symptoms.

12. A process for the preparation of a pharmaceutical comprising one or more of the compounds as claimed in one or more of claims 1 to 3, which comprises mixing the active compound with a pharmaceutically suitable excipient and bringing this mixture into a form suitable for administration.

13. The use of a compound as claimed in one or more of claims 1 to 3 for the preparation of a pharmaceutical for prophylaxis or treatment of disturbances in lipid metabolism.

14. The use of a compound as claimed in one or more of claims 1 to 3 for the preparation of a pharmaceutical for treatment of hyperlipidemia.

## Revendications

1. Composés de formule I 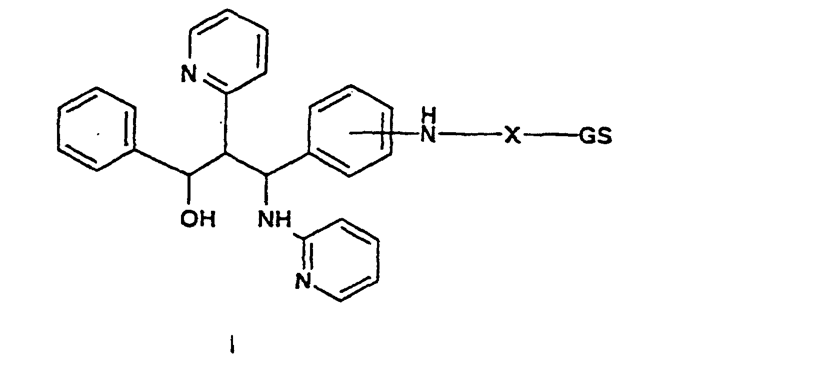 où
GS représente un groupe acide gallique de formule 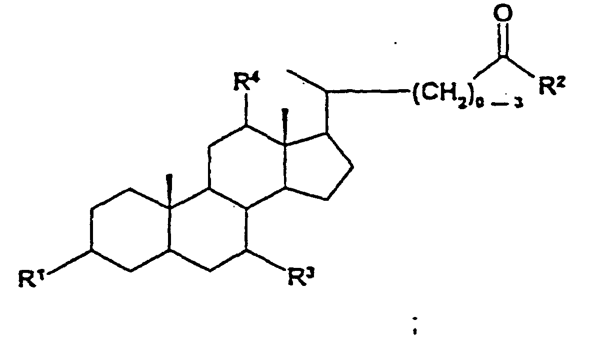
R¹ représente une liaison à X, un groupe OH ;
R² représente une liaison à X, un groupe OH, O-alkyle en C₁-C₆, NH- (alkyl en C₂-C₆)-SO₃H, N(CH₃)-CH₂-CH₂-SO₃H, NH- (alkyl en C₁-C₆)-COOH ; N(CH₃)-(alkyl en C₁-C₆)-COOH ;
à condition que R¹ et R² ne possèdent pas en même temps la signification suivante
R¹ une liaison à X et
R² une liaison à X ;
R³, R⁴ indépendamment l'un de l'autre représentent un atome d'hydrogène, un groupe OH ;
X 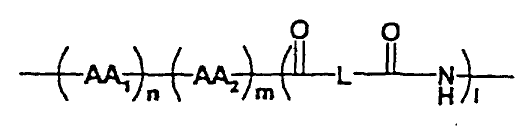 ou une liaison ;
l, m, n indépendamment les uns des autres valent 0 ou 1 ;
L représente un groupe alkyle en C₁-C₆, phényle ;
AA₁, AA₂ indépendamment l'un de l'autre représentent un reste acide aminé ou un reste acide aminé, qui est substitué une ou plusieurs fois par un groupe protecteur d'amino ;
ainsi que leurs sels tolérés en pharmacie.

2. Composés de formule I, selon la revendication 1, **caractérisés en ce que**, à cette formule
GS représente un groupe acide gallique de formule 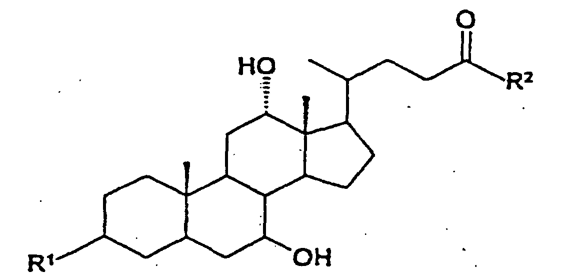
R¹ représente une liaison à X, un groupe OH ;
R² représente une liaison à X, un groupe OH, O-alkyle en C₁-C₆, NH- (alkyl en C₂-C₆)-SO₃H, N(CH₃)-CH₂-CH₂-SO₃H, NH-(alkyl en C₁-C₆)-COOH ; N(CH₃)-(alkyl en C₁-C₆)-COOH ;
à condition que R¹ et R² ne possèdent pas en même temps la signification suivante
R¹ une liaison à X et
R² une liaison à X ;
X 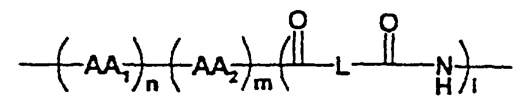 ou une liaison ;
1, m, n indépendamment les uns des autres valent 0 ou 1 ;
L représente un groupe alkyle en C₁-C₆, phényle ;
AA₁, AA₂ indépendamment l'un de l'autre représentent un reste acide aminé ou un reste acide aminé, qui est substitué une ou plusieurs fois par un groupe protecteur d'amino ;
ainsi que leurs sels tolérés en pharmacie.

3. Composés de formule I, selon la revendication 1 ou 2, **caractérisés en ce que**, à cette formule
GS représente un groupe acide gallique de formule 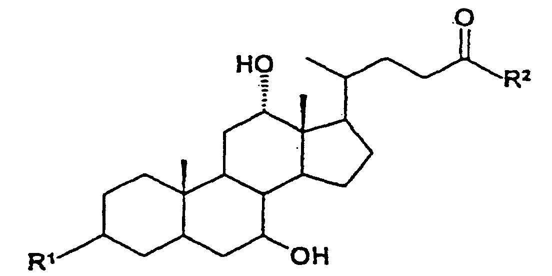
R¹ représente une liaison à X, un groupe OH ;
R² représente une liaison à X, un groupe OH, O-alkyle en C₁-C₆, NH-(alkyl en C₂-C₆)-SO₃H, NH-(alkyl en C₁-C₆)-COOH ;
à condition que R¹ et R² ne possèdent pas en même temps la siqnification suivante
R¹ une liaison à X et
R² une liaison à X ;
X 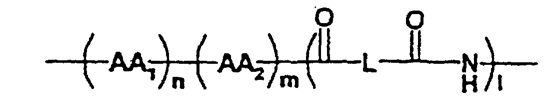 ou une liaison ;
l, m, n indépendamment les uns des autres valent 0 ou 1 ;
L représente un groupe alkyle en C₁-C₆ ;
AA₁, AA₂ indépendamment l'un de l'autre représentent un reste acide aminé ou un reste acide aminé qui est substitué une ou plusieurs fois par un groupe protecteur d'amino ;
ainsi que leurs sels tolérés en pharmacie.

4. Médicament contenant un ou plusieurs composés selon une ou plusieurs des revendications 1 à 3.

5. Médicament contenant un ou plusieurs composés selon une ou plusieurs des revendications 1 à 3 et un ou plusieurs principes actifs hypolipidémiants.

6. Composés selon une ou plusieurs des revendications 1 à 3 pour l'utilisation comme médicament pour la prévention ou le traitement de troubles de métabolisme lipidique.

7. Composés selon une ou plusieurs des revendications 1 à 3 pour l'application comme médicament pour le traitement d'hyperlipidémie.

8. Composés selon une ou plusieurs des revendications 1 à 3 pour l'application comme médicament pour la prévention ou le traitement de phénomènes artériosclérotiques.

9. Composés selon une ou plusieurs des revendications 1 à 3 en combinaison avec au moins un autre principe actif hypolipidémiant comme médicament pour la prévention ou le traitement de troubles de métabolisme lipidique.

10. Composés selon une ou plusieurs des revendications 1 à 3 en combinaison avec au moins un autre principe actif hypolipidémiant en tant que médicament pour la prévention ou le traitement d'hyperlipidémie.

11. Composés selon une ou plusieurs des revendications 1 à 3 en combinaison avec au moins un autre principe actif hypolipidémiant pour l'application comme médicament pour la prévention ou le traitement de phénomènes artériosclérotiques.

12. Procédé pour la préparation d'un médicament renfermant un ou plusieurs des composés selon une ou plusieurs des revendications 1 à 3, **caractérisé en ce qu'**on mélange le principe actif avec un véhicule approprié en pharmacie et on met ce mélange en forme d'administration appropriée.

13. Utilisation des composés selon une ou plusieurs des revendications 1 à 3 pour la préparation d'un médicament pour la prévention ou le traitement de troubles de métabolisme lipidique.

14. Utilisation des composés selon une ou plusieurs des revendications 1 à 3 pour la préparation d'un médicament pour le traitement d'hyperlipidémie.
